# EUROPÄISCHE PATENTSCHRIFT

(11) **EP 1 246 859 B1**
(45) Veröffentlichungstag und Bekanntmachung des Hinweises auf die Patenterteilung: **26.07.2006**
(21) Anmeldenummer: 01900445.6
(22) Anmeldetag: 15.01.2001
(51) Int. Cl.: C08G 59/00

(54) **POLYMERISIERBARE DENTALMASSEN AUF BASIS VON SILIZIUMHALTIGEN EPOXIDEN**
POLYMERIZABLE DENTAL COMPOSITIONS BASED ON EPOXIES THAT CONTAIN SILICON
COMPOSITIONS DENTAIRES POLYMERISABLES A BASE D'EPOXIDES CONTENANT DU SILICIUM

(30) Priorität: 13.01.2000 DE 10001228
(43) Veröffentlichungstag der Anmeldung: 09.10.2002
(73) Patentinhaber: 3M ESPE AG, 82229 Seefeld (DE)
(72) Erfinder: KLETTKE, Thomas, 86938 Schondorf (DE); WEINMANN, Wolfgang, 82205 Gilching (DE)
(86) Internationale Anmeldenummer: PCT/EP2001/000388
(87) Internationale Veröffentlichungsnummer: WO 2001/051540

(56) Entgegenhaltungen:
- EP-A- 0 897 710
- WO-A-00/19966
- WO-A-00/19967
- WO-A-98/33645
- DE-A- 19 648 283
- US-A- 5 639 413
- DATABASE WPI Section Ch, Week 198346 Derwent Publications Ltd., London, GB; Class A26, AN 1983-816671 XP002165315 & JP 58 168658 A (SHINETSU CHEM IND CO LTD), 5. Oktober 1983 (1983-10-05)

## Beschreibung

Die Erfindung betrifft polymerisierbare Zubereitungen auf der Basis von siliziumhaltigen Epoxiden und ihre Verwendung.

In polymerisierbaren Dentalmassen wurden bislang vorwiegend Methacrylat- und Acrylatmonomere verwendet. Besondere Aufmerksamkeit verdient das von Bowen beschriebene 2,2-Bis[4,1-phenylenoxy(2-hydroxy-3,1-propandiyl)-methacrylsäureester]-propyliden (Bis-GMA) (US-A-3 066 112). Mischungen dieses Methacrylats mit Triethylenglykoldimethacrylat dienen auch heute noch als Monomermatrix für dentale plastische Direkt-Füllungswerkstoffe. Auch Methacrylderivate des zweifach formylierten Bis-(hydroxymethyl)-tricyclo[5.2.1.0^{2,6}]-decans haben sich als Monomere für Dentalcomposite bewährt (W. Gruber et al., DE-A-27 14 538; W. Schmitt et al., DE-C-28 16 823; J. Reiners et al., EP-A-0 261 520). Ein großer Nachteil der bekannten polymerisierbaren Dentalmassen ist der Polymerisationsschrumpf, der beispielsweise bei der Anwendung als Füllungsmaterial durch die Bildung von Randspalten Sekundärkaries verursachen kann. Weiterhin führt bei Dentalmassen auf Acrylatbasis die Polymerisationsinhibierung durch Sauerstoff zur Ausbildung einer sogenannten Schmierschicht, die beispielsweise bei Füllungen unerwünscht bzw. sogar schädlich ist.

Obwohl es umfangreiche Erfahrungen mit Epoxiden und cycloaliphatischen Epoxiden gibt (US-A-2 716 123, US-A-2 750 395, US-A-2 863 881, US-A-3 187 018), sind solche Monomere und daraus formulierte kationisch polymerisierbare Massen mit den für dentale Anwendungen notwendigen Eigenschaften zu keinem Zeitpunkt kommerziell verfügbar gewesen.

Die Herstellung bifunktioneller cycloaliphatischer Epoxide ist bereits seit geraumer Zeit bekannt (US-A-2 750 395, US-A-900 506, US-A-907 149, US-A-2 745 847, US-A- 2 853, 499, US-A-3 187 018, US-A-2 863 881, US-A-2 853 498). Siliziumhaltige cycloaliphatische Epoxide zur Herstellung dreidimensionaler Objekte mittels Stereolithographie wurden von Crivello et al. in verschiedenen Publikationen beschrieben (WO 96/30182, EP-A-0 449 027; J.Polym.Sci., Part A: Polym.Chem. 28 (1990) 479, ibid. 31(1993)2563; ibid. 31 (1993) 2729; ibid. 31 (1993) 3109; ibid. 31 (1993) 3121: ibid. 33 (1995) 2463).

Es handelt sich bei den bekannten cycloaliphatischen Epoxiden im wesentlichen um niedermolekulare Monomere, die zwar einen verminderten Polymerisationsschrumpf besitzen (DE-A-4 340 949), die aber aufgrund ihrer toxikologischen Eigenschaften den Anforderungen an Werkstoffe für dentale Anwendungen nicht genügen.

Kationisch härtbare Epoxidmassen für dentale Anwendungen sind z.B. aus der US-A-5 556 896 bekannt. Diese Druckschrift beschreibt epoxidhaltige Massen, die notwendigerweise als schrumpfkompensierende Monomere Spiroorthocarbonate enthalten müssen.

Desweiteren werden in der WO 95/30402 photopolymerisierbare Verbindungen beschrieben, die Epoxidmonomere enthalten. Die dort beschriebenen Massen sind aufgrund ihrer hohen Wasseraufnahme im polymerisierten Zustand für dentale Anwendungen im Mundmilieu ungeeignet.

In den Schriften WO 98/47046, WO 98147047 und EP-A-0 897 710 werden Epoxidmassen für dentale Anwendungen beschrieben, die sich durch ein neues Initiatorsystem auszeichnen, aber auf herkömmlichen Epoxidmonomeren basieren. Die WO 98/22521 beschreibt polymerisierbare Massen auf der Basis von Epoxiden, unter anderem auch für die dentale Anwendung. Nachteilig an den dort offenbarten Epoxidmassen sind die relativ hohe Viskosität und die moderate Reaktivität der monomerhaitigen Massen.

Die bisher bekannten Epoxidmassen wiesen insbesondere bei der Verwendung von niederviskosen Monomeren hohe Toxizität und/oder Mutagenität auf, was die dentalen Anwendungsmöglichkeiten einschränkt.

Die Aufgabe der vorliegenden Erfindung ist es, Zubereitungen zur Verwendung als Dentalmasssen bereitzustellen, die sich durch gute Handlingseigenschaften, gute Verarbeitbarkeit, durch geringen Volumenschrumpf und hohe Reaktivität bei der Polymerisation sowie im polymerisierten Zustand durch eine hohe Stabilität und gute Biokompatibilität im Mundmilieu auszeichnen. Femer sollen die Monomere geringe Viskosität bei gleichzeitig geringer Toxizität und Mutagenität aufweisen.

Erfindungsgemäß wird diese Aufgabe gelöst durch polymerisierbare Dentalmassen, enthaltend
(a) 3 bis 80 Gew.-% eines Epoxids oder eines Gemisches von Epoxiden der allgemeinen Formel: 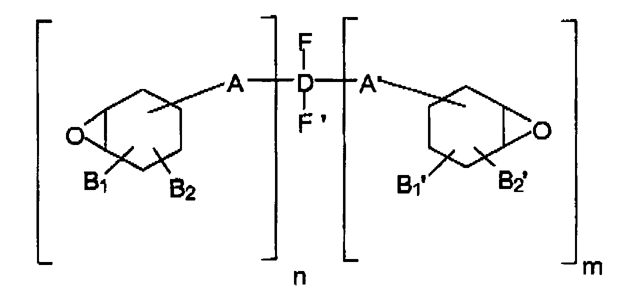 wobei
   A, A' unabhängig voneinander einen unverzweigten oder verzweigten aliphatischen, cycloaliphatischen oder aromatischen Rest mit 0 bis 13 C-Atomen bedeuten, wobei ein oder mehrere C-Atome durch, C=Q, O(C=O), Si, N, S ersetzt sein können,
   B1, B1', B2, B2' unabhängig voneinander H, einen unverzweigten oder verzweigten aliphatischen, cycloaliphatischen oder aromatischen Rest mit 0 bis 6 C-Atomen bedeuten, wobei ein oder mehrere C-Atome durch O, (C=O), O(C=O), Si, N, S ersetzt sein können,
   F, F' unabhängig voneinander einen unverzweigten oder verzweigten aliphatischen, cycloaliphatischen oder aromatischen Rest mit 0 bis 10 C-Atomen bedeuten, wobei ein oder mehrere C-Atome durch (C=O), O(C=O), Si, N, S ersetzt sein können,
   D einen der folgenden Bestandteile darstellt, der über das Si-Atom an die Bestandteile A und/oder A' angebunden ist:
      i. Si
      ii. SiG
      iii. SiGC'
      iv. 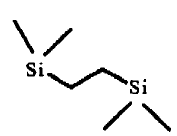
      v. 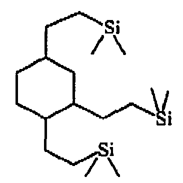
      vi. 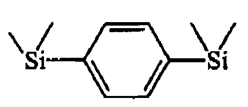
      vii. 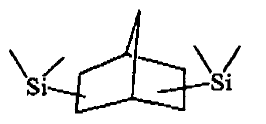
      wobei G, G' unabhängig voneinander einen unverzweigten oder verzweigten aliphatischen, cycloaliphatischen oder aromatischen Rest mit 0 bis 8 C-Atomen bedeuten, wobei ein oder mehrere C-Atome durch (C=O) O(C=O), si ersetzt sein können.
   G, G' unabhängig voneinander H, einen unverzweigten oder verzweigten aliphatischen, cycloaliphatischen oder aromatischen Rest mit 0 bis 8 C-Atomen oder eine Kombination dieser Reste bedeuten, wobei ein oder mehrere C-Atome durch, (C=O), O(C=O), ersetzt sein können,
   n und m unabhängig voneinander 0, 1, 2 oder 3 bedeuten und n+m 2 bis 6 ergeben
   und wobei die Molmasse des Epoxids oder die mittlere Molmasse des Gemisches von Epoxiden 250 bis 1000 g/mol beträgt,
(b) 0 bis 80 Gew.% eines Epoxids oder eines Gemisches von Epoxiden, die von (a) verschieden sind,
(c) 30 bis 85 Gew.-% Füllstoffe,
(d) 0,01 bis 25 Gew.-% Initiatoren, Verzögerer und/oder Beschleuniger,
(e) 0 bis 25 Gew.-% Hilfsstoffe,
   wobei die Prozentangaben jeweils auf das Gesamtgewicht der Zubereitung bezogen sind.

Bevorzugte polymerisierbare Dentalmassen enthalten
(a) 5 bis 50 Gew.-% eines Epoxids oder eines Gemisches von Epoxiden der allgemeinen Formel: 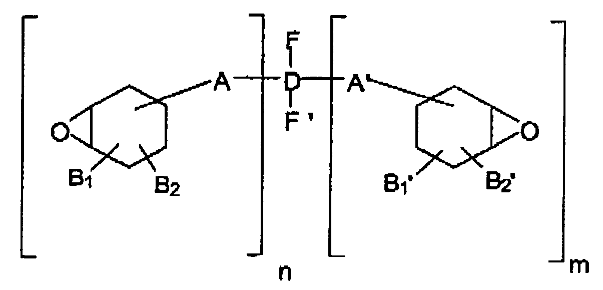 wobei
   A, A' unabhängig voneinander einen unverzweigten oder verzweigten aliphatischen, cycloaliphatischen oder aromatischen Rest mit 0 bis 10 C-Atomen bedeuten, wobei ein oder mehrere C-Atome durch C=O, O(C=O), Si ersetzt sein können,
   B1. B1', B2, B2" unabhängig voneinander H, einen unverzweigten oder verzweigten aliphatischen Rest mit 0 bis 4 C-Atomen bedeuten, wobei ein oder mehrere C-Atome durch O, (C=O), O(C=O), Si ersetzt sein können,
   F, F' unabhängig voneinander einen unverzweigten oder verzweigten aliphatischen, cycloaliphatischen oder aromatischen Rest mit 0 bis 10 C-Atomen bedeuten, wobei ein oder mehrere C-Atome durch (C=O), O(C=O), Si ersetzt sein können,
   D einen der folgenden Bestandteile darstellt, der über das Si-Atom an die Bestandteile A und /oder A' angebunden ist:
      i. Si
      ii. SiG
      iii. SiGG'
      iv. 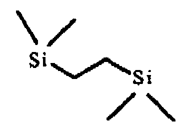
      v. 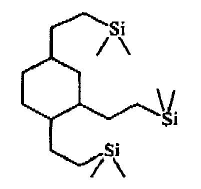
      vi. 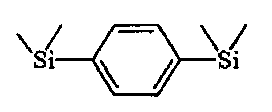
      vii. 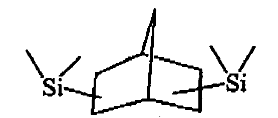
      wobei G, G' unabhängig voneinander einen unverzweigten oder verzweigten aliphatischen, cycloaliphatischen oder aromatischen Rest mit 0 bis 8 C-Atomen bedeuten, wobei ein oder mehrere C-Atome durch (C=O), O(C=O) Si ersetzt sein können,
   n und m unabhängig voneinander 0, 1, 2 oder 3 bedeuten und n+m 2 bis 6 ergeben,
   und wobei die Molmasse des Epoxids oder die mittlere Molmasse des Gemisches von Epoxiden 250 bis 1000 g/mol beträgt,
(b) 0 bis 60 Gew.-% eines Epoxids oder eines Gemisches von Epoxiden, die von (a) verschieden sind,
(c) 30 bis 85 Gew.-% Füllstoffe,
(d) 0,01 bis 20 Gew.-% Initiatoren, Verzögerer und/oder Beschleuniger,
(e) 0 bis 25 Gew.-% Hilfsstoffe,
   wobei die Prozentangaben jeweils auf das Gesamtgewicht der Zubereitung bezogen sind.

Durch den Einsatz von Epoxiden bzw. eines Epoxidgemischs nach Komponente (a) mit einer mittleren Molmasse von 250 bis 1000 g/mol wird erreicht, dass die erfindungsgemäßen Dentalmassen eine bessere Verarbeitbarkeit und verbesserte Handlingseigenschaften aufweisen. Dies beruht insbesondere auf einer geringen Viskosität der Komponente (a) in den erfindungsgemäßen Zubereitungen. Besonders vorteilhaft sind dabei Epoxide bzw. ein Epoxidgemisch nach Komponente (a) mit einer mittleren Molmasse von 250 bis 500 g/mol.

Überraschend wurde gefunden, dass diese niedrigviskosen Epoxide bzw. Epoxidgemische nur eine geringe Mutagenität aufweisen.

Überraschend wurde festgestellt, dass die erfindungsgemäße polymerisierbare Dentalmasse dann besonders gute bioverträgliche Eigenschaften aufweist, wenn sie als Komponente (a) eines oder mehrere der folgenden Epoxide enthält: 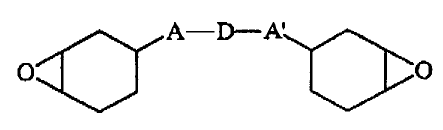 wobei
A, A" unabhängig voneinander einen aliphatischen Rest mit 0 bis 2 C-Atomen bedeuten,
D SiGG' bedeutet,
G, G' unabhängig voneinander einen unverzweigten oder verzweigten aliphatischen oder aromatischen Rest mit 0 bis 8 C-Atomen bedeuten,
und wobei die Molmasse des Epoxids oder die mittlere Molmasse des Gemisches von Epoxiden 250 bis 500 g/mol beträgt,

Zu besonders guten Ergebnissen führen Dentalmassen die eines oder mehrere der folgenden Epoxide enthalten, die im folgenden nach der gültigen IUPAC-Nomenklatur benannt sind:
i. Silane, methylbis[2-(7-oxabicyclo[4.1.0]hept-3-yl)ethyl]phenyl-(CAS-Nr. 154265-59-5) 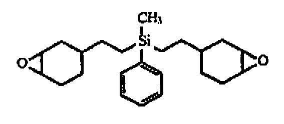
ii. Silane, dimethylbis[2-(7-oxabicyclo[4.1.0]hept-3-yl)methyl]- 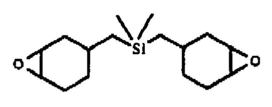
iii. Silane, dimethyl[2-(7-oxabicyclo[4.1.0]hept-3-yl)methyl] [2-(7-oxabicyclo[4.1.0]hept-3-yl)ethyl]- 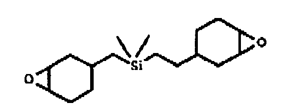
iv. Silane, 1,4-phenylenbis[dimethyl[2-(7-oxabicyclo[4.1.0]hept-3-yl)ethyl]]- (CAS-Nr. 20988-18-8) 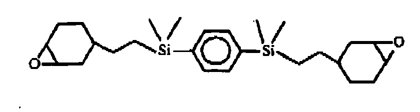
v. Silane, 1,2-ethylenbis[dimethyl[2-(7- oxabicyclo[4.1.0]hept-3-yl)ethyl]]- 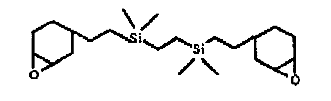
vi. Silane, dimethylbis[2-(7-oxabicyclo[4.1.0]hept-3-yl)ethyl]- 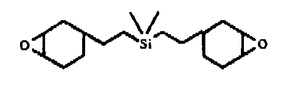
vii. Silane, 2,5-bicyclo[2.2.1.]heptylenbis[dimethyl[2-(7-oxabicyclo[4.1.0]hept-3-yl)ethyl]]- 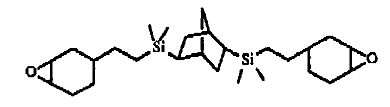
viii. Silane, 1,6-hexylenbis[dimethyl[2-(7-oxabicyclo[4.1.0]hept-3-yl)ethyl]]- 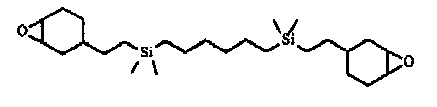
ix. Silane, 1,1',1"-(1,2,4-cyclohexylentris(dimethyl[2-(7-oxabicyclo[4.1.0]hept-3-yl)ethyl])) -

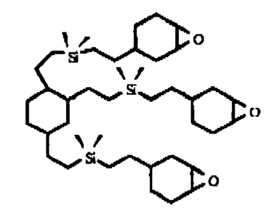

Die erfindungsgemäßen polymerisierbaren Dentalmassen können neben den beschriebenen siliziumhaltigen Epoxiden als Komponente (b) andere Epoxide enthalten. Epoxide gemäß (b) können beispielsweise sein: 3,4-Epoxycyclohexylmethyl-3,4-epoxycyclohexancarboxylat (US-A-2 716 123), 3,4-Epoxy-6-methylcyclohexyl-3,4-epoxy-6-methylcyclohexancarboxylat (US-A-2 716 123) oder verwandte Epoxide, Vinylcyclohexendiepoxid (US-A-2 948 688), Dicyclopentadiendioxid (US-A-2 985 667), Bis(3,4-epoxycyclohexylmethyl)adipat (US-A-2 750 395, US-A-2 863 881, US-A-3 187 018),
7-Oxabicyclo[4.1.0]heptane, 3,3',3",3"'-[(2,4,6,8-tetramethylcyclotetrasiloxan-2,4,6,8-tetrayl)tetra-2,1-ethandiyl]tetrakis- der folgenden Formel: 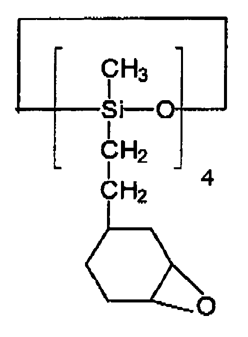 7-Oxabicyclo[4.1.0]heptan, 3,3',3",3"',3""-[(2,4,6,8,10-pentamethylcyclopentasiloxan-2,4,6,8,10-pentayl)penta-2,1-ethandiyl]pentakis- der folgenden Formel:

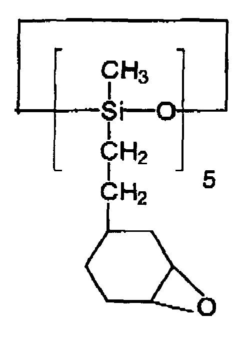

Die Epoxide gemäß Komponente (b) können in einer Konzentration von 0 bis 80 Gew.-%, vorzugsweise 0 bis 60 Gew.-%, vorhanden sein, jeweils bezogen auf das Gesamtgewicht der Zubereitung.

Anorganische Füllstoffe gemäß Komponente (c) können übliche dentale Füllstoffe, beispielsweise Quarz, gemahlene, gegebenenfalls röntgenopake, gegebenenfalls reaktive Gläser, schwer lösliche Fluoride, wie CaF₂, YF₃ (EP-B-0 238 025), Kieselgele sowie pyrogene Kieselsäure und/oder deren Granulate sein.

Ebenso können ein oder mehrere wasserlösliche anorganische komplexe Fluoride der allgemeinen Formel AₙMFₘ, worin A ein ein- oder mehrwertiges Kation, M ein Metall der III, IV oder V Haupt-oder Nebengruppe, n eine ganze Zahl von 1 bis 3 und m eine ganze Zahl von 4 bis 6 bedeuten (DE-A-4 445 266), als fluoridabgebende Bestandteile in der Komponente (c) enthalten sein. Sie können in einer Konzentration von 30 bis 85 Gew.-%, bezogen auf die Gesamtmasse, in den polymerisierbaren Dentalmassen enthalten sein.

Zum besseren Einbau in die Polymermatrix kann es von Vorteil sein, die genannten Füllstoffe durch übliche Verfahren zu hydrophobieren. Übliche Hydrophobierungsmittel sind Silane, beispielsweise Trimethoxyglycidylsilan. Die mittlere Komgröße der anorganischen Füllstoffe beträgt vorzugsweise <20 µm, insbesondere <12 µm. Ganz besonders bevorzugt werden Füllstoffe mit einer mittleren Korngröße < 7 µm eingesetzt.

Auch Christobalit, Calciumsilikat, Zirkoniumsilikat, Mondmorillonite wie Bentonite, Zeolithe, einschließlich der Molekularsiebe, wie Natriumaluminiumsilikat, Metalloxidpulver, wie Aluminium- oder Zinkoxide bzw. deren Mischoxide, Bariumsulfat, Calciumcarbonat, Gips und Kunststoffpulver sind als Füllstoffe geeignet.

Initiatoren gemäß Komponente (d) der erfindungsgemäßen Zubereitungen können sein: Lewis- oder Broensted-Säuren bzw. Verbindungen, die solche Säuren freisetzen, welche die Polymerisation initiieren, beispielsweise BF₃ oder dessen etherische Addukte (BF₃.THF, BF₃.Et₂O, etc.), AlCl₃, FeCl₃, HPF₆, HAsF₆, HSbF₆, HBF₄ oder Substanzen, die nach Bestrahlen durch UV, sichtbares Licht, Wärme und/oder Druck die Polymerisation auslösen, wie z.B. (eta-6-Cumol)(eta-5-cyclopentadienyl)eisenhexafluorophosphat, (eta-6-Cumol)(eta-5-cyclopentadienyl)eisen-tetrafluoroborat, (eta-6-Cumol)(eta-5-cyclopentadienyl)eisenhexafluoroantimonat, substituierte Diaryliodoniumsalze und Triarylsulfoniumsalze. Als Beschleuniger können Peroxyverbindungen vom Typ der Perester, der Diacylperoxide, der Peroxydicarbonate und der Hydroperoxide eingesetzt werden. Bevorzugt werden Hydroperoxide verwendet und besonders bevorzugt kommt als Beschleuniger Cumolhydroperoxid in etwa 70 bis 90 %iger Lösung in Cumol zum Einsatz. Das Verhältnis von Photoinitiator zu Cumolhydroperoxid kann in weiten Grenzen von 1:0,001 bis 1:10 variiert werden, vorzugsweise wird jedoch ein Verhältnis von 1:0,1 bis 1:6 und besonders bevorzugt von 1:0,5 bis 1:4 verwendet. Die Verwendung von Komplexbildnern, wie beispielsweise Oxalsäure, 8-Hydroxychinolin, Ethylendiamintetraessigsäure und aromatischen Polyhydroxyverbindungen ist ebenfalls möglich.

Ebenso können Initiatorsysteme bestehend aus verschiedenen Komponenten eingesetzt werden, wie sie in EP 0 897 710 A2, WO 98/47046 oder WO 98/47047 beschrieben sind. Bevorzugt werden Initiatorsysteme bestehend aus 1,2-Diketonen, wie Campherchinon, lodoniumiumsalze mit wenig koordinierenden Anionen, wie Tolylcumyliodonium tetrakis-(pentafluorophenyl)borat und aromatische tertiäre Amine, 2-Butoxyethyl-4-(dimethylamino)benzoat oder Ethyl-4-(dimethylamino)benzoat eingesetzt.

Als Verzögerer können Basen, typischerweise tertiäre Amine, zugesetzt werden. Die Komponente (d) liegt in den erfindungsgemäßen Zubereitungen in einer Menge von 0,01 bis 25 Gew.-%, vorzugsweise 0,01 bis 20 Gew.-%, bezogen auf das Gesamtgewicht der Zubereitung, vor.

Geeignete Hilfsstoffe gemäß Komponente (e) können beispielsweise üblicherweise auf dem Dentalgebiet eingesetzte Stabilisatoren (z.B. Tinuvine der Fa. Ciba), Pigmente oder Verdünnungsmittel sein.

Überraschenderweise wurde gefunden, dass die siliziumhaltigen Epoxide oder Gemische gemäß Komponente (a) der erfindungsgemäßen Dentalmassen bei vorteilhaft niedriger Viskosität bessere Toxizitätsdaten aufweisen als ähnliche niederviskose Epoxide bereits beschriebener Dentalmassen. Die verbesserte Toxizität äußert sich beispielsweise in einer niedrigeren Mutagenität. Polymerisierbare Massen mit Epoxide oder Gemischen von Epoxiden gemäß Komponente (a), die mindestens ein Si-Atom im Rest D der Moleküle aufweisen, zeigen unerwarteterweise eine niedrigere Mutagenität als vergleichbare Epoxide ohne Si-Atom im Rest D der Moleküle. Vergleichsversuche zeigen, dass die siliziumhaltigen Epoxide der beanspruchten Zubereitungen im Mutagenitätstest (Ames Test, ISO/FDIS 7405) bessere Ergebnisse liefern als beispielsweise aus dem Stand der Technik bekannte 3,4-Epoxycyclohexylmethyl-3,4-epoxycyclohexancarboxylat.

| Bezeichnung nach IUPAC | Ames-Test (ISO/FDIS 7405) |
|---|---|
| Silane, methyl bis[2-(7-oxabicyclo[4.1.0]hept-3-yl)ethyl]phenyl | Negativ |
| Silane, dimethylbis [2-(7-oxabicyclo[4.1.0]hept-3-yl)methyl] | Negativ |
| Epoxycyclohexylmethyl-3,4-epoxycyclohexancarboxylat (Vergleich) | Positiv |

Die erfindungsgemäßen epoxidhaltigen, polymerisierbaren Zubereitungen eignen sich insbesondere als Werkstoffe für dentale Zwecke, beispielsweise zur Herstellung von Kunststoffzähnen oder Provisorien, als Beschichtungsmittel, zum Verkleben von Substraten sowie als dentale Füllungsmaterialien. Dabei ist z.B. eine Beschichtung von Kunststoffen, Gläsern, Papier, Folien, Metallen oder mineralischen Substraten möglich.

Die polymerisierbare Dentalmasse kann als einkomponentiges System zur Verfügung gestellt werden. Ebenso ist eine Formulierung als zwei- oder mehrkomponentiges System denkbar. Dabei können eine oder mehrere Basispasten (A) Epoxide oder Gemische von Epoxiden der Komponenten (a) und (b), einen Teil oder den gesamten Anteil der Füllstoffe der Komponente (c), gegebenenfalls Verzögerer und/oder Beschleuniger gemäß Komponente (d) und gegebenenfalls Hilfsstoffe der Komponente (e) enthalten. Räumlich getrennt hiervon können eine oder mehrere Katalysatorpasten (B) einen oder mehrere Initiatoren gemäß Komponente (d), gegebenenfalls Verzögerer und/oder Beschleuniger gemäß Komponente (d) gegebenenfalls einen Teil der Füllstoffe der Komponente (c) und gegebenenfalls Hilfsstoffe gemäß Komponente (e) aufweisen. Die Pasten (A) und (B) werden dann zum Erhalt der polymerisierbaren Zubereitung miteinander zur Reaktion gebracht. Dies geschieht beispielsweise durch automatisches oder manuelles Mischen von Basis- und Katalysatorpasten.

Die erfindungsgemäße Dentalmasse kann in verschiedene Behältnisse abgepackt werden. Geeignet sind z.B. Kartuschen mit einer oder mehreren Kammern, Mischkapseln, Schraubtuben oder Tuben. Femer kann die polymerisierbare Zubereitung in verschiedenen Ausbringvorrichtungen enthalten sein.

In nachstehender Tabelle sind Beispiele für Monomerzusammensetzungen genannt, die die Aufgabe der vorliegenden Erfindung lösen. Die Biegefestigkeit und die Wasseraufnahme wurde gemäß ISO 4049 bestimmt. Der Volumenschrumpf wurde aus den archimedisch bestimmten Dichten und Volumina der unpolymerisierten und der polymerisierten Zubereitungen errechnet.

Die Monomere bzw. Monomermischungen gemäß Komponente (a) wiesen eine alle eine Viskosität kleiner als 3 Pas auf. Die Viskosität wurde kraftgesteuert bestimmt und die Epoxide bzw. die Epoxidmischungen gemäß Komponente (a) im Meßbereich von 5-500 Pa als Newtonsch'sche Flüssigkeiten erkannt. Die aus dem Stand der Technik bekannten Monomere bzw. Monomermischungen haben unter diesen Meßbedingungen bei vergleichbarer Toxizität und Mutagenität Viskositäten größer als 20 Pas. unter diesen Meßbedingungen bei vergleichbarer Toxizität und Mutagenität Viskositäten größer als 20 Pas.

**Monomerzusammensetzungen**

| Anteile in Gew.-% | Monomerzusammensetzung 1 | Monomerzusammensetzung 2 | Monomerzusammensetzung 3 | Monomerzusammensetzung 4 | Monomerzusammensetzung 5 | Monomerz sammensetzung 6 |
|---|---|---|---|---|---|---|
| Silane, methyl bis[2-(7- oxabicyclo[4.1.0]hept-3-yl)ethyl]phenyl | 60 | 40 | | | | 100 |
| Silane, 1,4-phenylen bis[dimethyl[2-(7- oxabicyclo[4.1.0]hept-3-yl)ethyl]]- | 40 | | 33 | | 30 | |
| Silane, dimethylbis [2-(7- oxabicyclo[4.1.0]hept-3-yl)methyl] | | | 33 | 50 | | |
| Disiloxane, 1,1',1 "-(1,2,4-cyclohexanetriyltri- 2,1-ethanediyl)tris[1,1,3,3-tetramethyl-3-[2-(7-oxabicyclo[4.1.0]hept-3-yl)ethyl] | | | | | 40 | |
| 1,3,5,7-Tetrakis(2,1-ethandiyl-3,4- epoxycyclohexyl)-1,3,5,7-tetramethylcyclotetrasiloxan | | 60 | 34 | 50 | 30 | |

**Erfindungsgemäße Zusammensetzungen mit Initiatoren und Füllstoffen sowei deren Biegefestigkeit, Wasseraufnahme und Volumenschrumpf**

| Anteile in Gew.-% | Beispiel 1 | Beispiel 2 | Beispiel 3 | Beispiel 4 | Beispiel 5 | Beispiel 6 | Beispiel 7 | Beispiel 8 |
|---|---|---|---|---|---|---|---|---|
| Monomerzusammensetzung 1 | 36,0 | | | | | | | |
| Monomerzusammensetzung 2 | | 18,0 | 23,3 | | | | | |
| Monomerzusammensetzung 3 | | | | 20,5 | | | | |
| Monomerzusammensetzung 4 | | | | | 19,0 | 41,0 | | |
| Monomerzusammensetzung 5 | | | | | | | 20,4 | |
| Monomerzusammensetzung 6 | | | | | | | | 20,0 |
| Tolylcumyliodonium tetrakis(pentafluorophenyl)borat | 2,1 | 2,0 | 2,2 | 2,1 | 2,1 | 2,4 | 2,1 | 2,2 |
| 2-Butoxyethyl-4-(dimethylamiono)benzoat | 0,3 | 0,2 | 0,2 | 0,2 | 0,2 | 0,3 | 0,2 | 0,3 |
| Campherchinon | 0,6 | 0,5 | 0,6 | 0,5 | 0,6 | 0,7 | 0,5 | 0,6 |
| Quarz | | 79,3 | | | 78,1 | | | |
| Schott Glas GM 27884 | 61,0 | | 73,7 | 76,66 | | 55,6 | 76,8 | 76,9 |
| Tinuvin P | | | | 0,04 | | | | |
| Biegefestigkeit (ISO4049) [MPa] | 89 | 121 | 116 | 102 | 118 | 93 | 97 | 108 |
| Wasseraufnahme [µg/mm³]] | 10,8 | 6,3 | 8,1 | 7,8 | 7,6 | 19,1 | 7,7 | 6,4 |
| Volumenschrumpf [Vol-%] | 1,7 | 1,1 | 1,4 | 1,2 | 1,0 | 1,9 | 1,4 | 1,4 |

## Patentansprüche

1. Polymerisierbare Dentalmasse enthaltend:
(a) 3 bis 80 Gew.-% eines Epoxids oder eines Gemisches von Epoxiden der allgemeinen Formel: 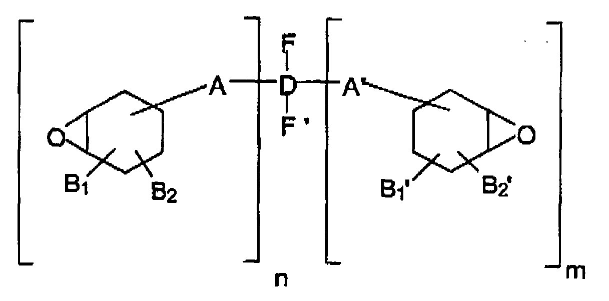 wobei
A, A' unabhängig voneinander einen unverzweigten oder verzweigten aliphatischen, cycloaliphatischen oder aromatischen Rest mit 0 bis 13 C-Atomen bedeuten, wobei ein oder mehrere C-Atome durch C=O, O(C=O), Si, N. S ersetzt sein können,
B1, B1', B2, B2' unabhängig voneinander H, einen unverzweigten oder verzweigten aliphatischen, cycloaliphatischen oder aromatischen Rest mit 0 bis 6 C-Atomen bedeuten, wobei ein oder mehrere C-Atome durch O, (C=O), O(C=O), Si, N, S ersetzt sein können,
F, F' unabhängig voneinander einen unverzweigten oder verzweigten aliphatischen, cycloaliphatischen oder aromatischen Rest mit 0 bis 10 C-Atomen bedeuten, wobei ein oder mehrere C-Atome durch (C=O), O(C=O), Si, N, S ersetzt sein können,
D einen der folgenden Bestandteile darstellt der über das Si-Atom an die Bestandteile A und /oder A' angebunden ist:
i. Si
ii. SiG
iii. SiGG'
iv. 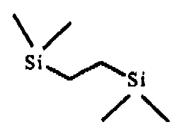
v. 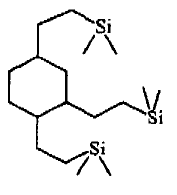
vi. 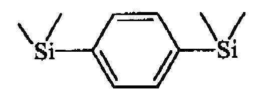
vii. 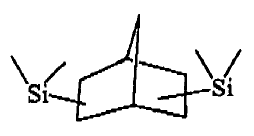
wobei G, G' unabhängig voneinander einen unverzweigten oder verzweigten aliphatischen, cycloaliphatischen oder aromatischen Rest mit 0 bis 8 C-Atomen bedeuten, wobei ein oder mehrere C-Atome durch (C=O), O(C=O), Si ersetzt sein können,
n und m unabhängig voneinander 0, 1, 2 oder 3 bedeuten und n+m 2 bis 6 ergeben,
und wobei die Molmasse des Epoxids oder die mittlere Molmasse des Gemisches von Epoxiden 250 bis 1000 g/mol beträgt,
(b) 0 bis 80 Gew.-% eines Epoxids oder eines Gemisches von Epoxiden, die von (a) verschieden sind,
(c) 30 bis 85 Gew.-% Füllstoffe,
(d) 0,01 bis 25 Gew.-% Initiatoren, Verzögerer und/oder Beschleuniger,
(e) 0 bis 25 Gew.-% Hilfsstoffe,
wobei die Prozentangaben jeweils auf das Gesamtgewicht der Zubereitung bezogen sind.

2. Polymerisierbare Dentalmasse nach Anspruch 1, enthaltend:
(a) 5 bis 50 Gew.-% eines Epoxids oder eines Gemisches von Epoxiden der allgemeinen Formel: 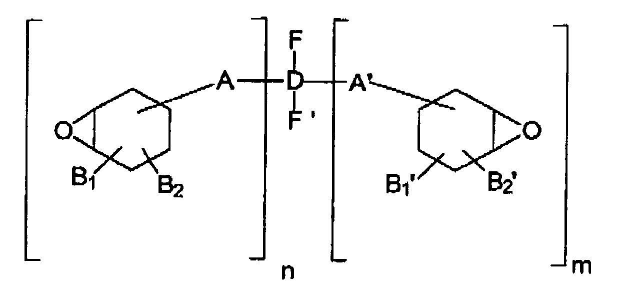 wobei
A, A' unabhängig voneinander einen unverzweigten oder verzweigten aliphatischen, cycloaliphatischen oder aromatischen Rest mit 0 bis 10 C-Atomen bedeuten, wobei ein oder mehrere C-Atome durch C=O, O(C=O), Si ersetzt sein können,
B1. B1', B2, B2' unabhängig voneinander H, einen unverzweigten oder verzweigten aliphatischen Rest mit 0 bis 4 C-Atomen bedeuten, wobei ein oder mehrere C-Atome durch O, (C=O), O(C=O), Si ersetzt sein können,
F, F' unabhängig voneinander einen unverzweigten oder verzweigten aliphatischen, cycloaliphatischen oder aromatischen Rest mit 0 bis 10 C-Atomen bedeuten, wobei ein oder mehrere C-Atome durch (C=O), O(C=O), Si ersetzt sein können,
D einen der folgenden Bestandteile darstellt, der über das Si-Atom an die Bestandteile A und /oder A' angebunden ist:
i. Si
ii. SiG
iii. SiGG'
iv. 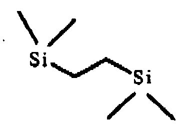
v. 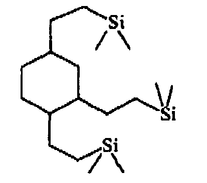
vi. 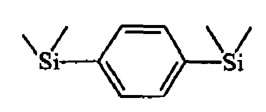
vii. 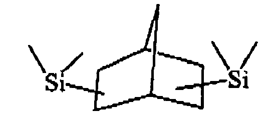
wobei G, G' unabhängig voneinander einen unverzweigten oder verzweigten aliphatischen, cycloaliphatischen oder aromatischen Rest mit 0 bis 8 C-Atomen bedeuten, wobei ein oder mehrere C-Atome durch (C=O), O(C=O), Si ersetzt sein können,
n und m unabhängig voneinander 0, 1, 2 oder 3 bedeuten und n+m 2 bis 6 ergeben,
und wobei die Molmasse des Epoxids oder die mittlere Molmasse des Gemisches von Epoxiden 250 bis 1000 g/mol beträgt,
(b) 0 bis 60 Gew.-% eines Epoxids oder eines Gemisches von Epoxiden, die von (a) verschieden sind,
(c) 30 bis 85 Gew.-% Füllstoffe,
(d) 0,01 bis 20 Gew.-% Initiatoren, Verzögerer und/oder Beschleuniger,
(e) 0 bis 25 Gew.-% Hilfsstoffe,
wobei die Prozentangaben jeweils auf das Gesamtgewicht der Zubereitung bezogen sind.

3. Polymerisierbare Dentalmasse nach einem der Ansprüche 1 bis 2, wobei sie als Komponente (a) eines oder mehrere der folgenden Epoxide enthält: 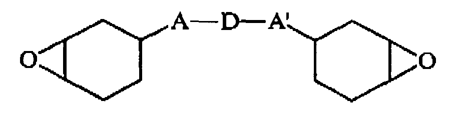 wobei
A, A' unabhängig voneinander einen aliphatischen Rest mit 0 bis 2 C-Atomen bedeuten,
D SiGG' bedeutet,
G, G' unabhängig voneinander einen unverzweigten oder verzweigten aliphatischen oder aromatischen Rest mit 0 bis 8 C-Atomen bedeuten,
und wobei die Molmasse des Epoxids oder die mittlere Molmasse des Gemisches von Epoxiden 250 bis 500 g/mol beträgt.

4. Polymerisierbare Dentalmasse nach Anspruch 1 bis 2, wobei sie als Komponente (a) eines oder mehrere der folgenden Epoxide enthält:
i. Silane, methylbis[2-(7-oxabicyclo[4,1.0]hept-3-yl)ethyl]phenyl- 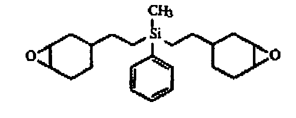
ii. Silane, dimethylbis[2-(7-oxabicyclo[4.1.0]hept-3-yl)methyl]- 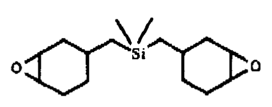
iii. Silane, dimethyl[2-(7-oxabicyclo[4.1.0]hept-3-yl)methyl] [2-(7-oxabicyclo[4.1,0]hept-3-yl)ethyl]- 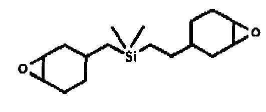
iv. Silane, -1,4-phenylenbis[dimethyl[2-(7-oxabicyclo[4.1.0]hept-3-yl)ethyl]- 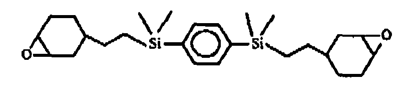
v. Silane, 1,2-ethylenbis[dimethyl[2-(7- oxabicyclo[4.1.0]hept-3-yl)ethyl]]- 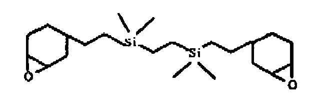
vi. Silane, dimethylbis[2-(7-oxablcyclo[4.1.0]hept-3-yl)ethyl]- 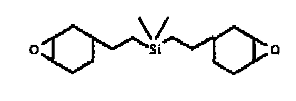
vii. Silane, -2,5-bicyclo[2.2.1.]heptylenbis[dimethyl[2-(7-oxabicyclo[4.1.0]hept-3-yl]ethyl]- 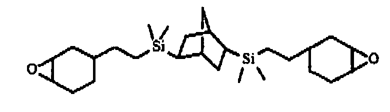
viii. Silane, 1,6-hexylenbis[dimethyl[2-(7-oxabicyclo[4.1.0]hept-3-yl)ethyl]- 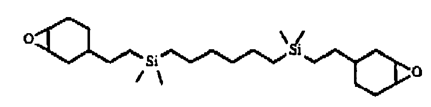
ix. Silane, 1,1',1"-(1,2,4-cyclohexantriyltri-2,1-ethanediyl)tris[dimethyl-[2-(7-oxabicyclo [4.1.0]hept-3-yl)ethyl]]-

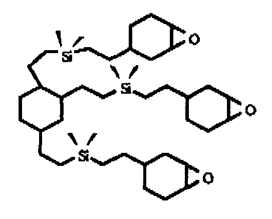

5. Polymerisierbare Dentalmasse nach einem der Ansprüche 1 bis 4 , wobei sie als Füllstoffe gemäß Komponente (c) Quarz, gemahlene Gläser, Kieselgele und/oder Kieselsäuren, deren Granulate und/oder gemahlene Kunststoffe enthält.

6. Polymerisierbare Dentalmasse nach einem der Ansprüche 1 bis 5, wobei sie als Initiatoren Lewis-Säuren und/oder Brönsted-Säuren oder Verbindungen, aus denen durch Bestrahlen mit UV-Licht, sichtbarem Licht, Druck und/oder Wärme oder durch chemische Reaktion solche Säuren entstehen, enthält.

7. Polymerisierbare Dentalmasse nach einem der Ansprüche 1 bis 6 , wobei sie als Hilfsstoffe Verdünnungsmittel, Stabilisatoren, Inhibitoren und/oder Pigmente enthält.

8. Polymerisierbare Dentalmasse nach einem der Ansprüche 1 bis 7, enthaltend
A mindestens eine Basispaste, enthaltend Epoxide oder Gemische von Epoxiden der Komponenten (a) und (b), einen Teil oder den gesamten Anteil der Füllstoffe der Komponente (c), gegebenenfalls Verzögerer und/oder Beschleuniger gemäß Komponente (d), und gegebenenfalls Hilfsstoffe der Komponente (e), sowie räumlich getrennt hiervon
B mindestens eine Katalysatorpaste, enthaltend mindestens einen Initiator gemäß Komponente (d) gegebenenfalls einen Teil der Füllstoffe der Komponente (c) und gegebenenfalls Hilfsstoffe gemäß Komponente (e),
wobei Basis- und Katalysatorpaste zum Erhalt der polymerisierbaren Dentalmasse miteinander zur Reaktion gebracht werden.

9. Behältnis, insbesondere Kartusche oder Mischkapsel, enthaltend eine polymerisierbare Dentalmasse nach einem der Ansprüche 1 bis 8 .

10. Ausbringvorrichtung, enthaltend eine polymerisierbare Dentalmasse nach einem der Ansprüche 1 bis 8**.**

## Claims

1. Polymerizable dental material comprising:
(a) 3 to 80 weight% of an epoxide or a mixture of epoxides of the general formula: 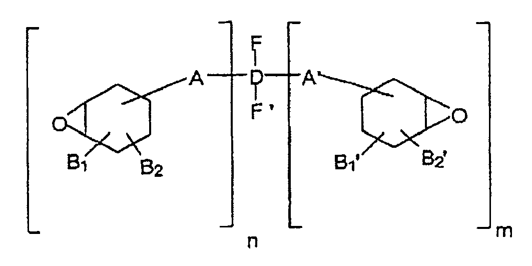 in which
A and A' represent, independently of one another, an unbranched or branched aliphatic, cycloaliphatic or aromatic residue with 0 to 13 carbon atoms, in which one or more carbon atoms can be replaced by C=O, O(C=O), Si, N or S,
B1, B1', B2 and B2' represent, independently of one another, H or an unbranched or branched aliphatic, cycloaliphatic or aromatic residue with 0 to 6 carbon atoms, in which one or more carbon atoms can be replaced by O, (C=O), O(C=O), Si, N or S,
F and F' represent, independently of one another, an unbranched or branched aliphatic, cycloaliphatic or aromatic residue with 0 to 10 carbon atoms, in which one or more carbon atoms can be replaced by (C=O), O(C=O), Si, N or S,
D represents one of the following constituents, which is bonded to the constituents A and/or A' via the Si atom:
i. Si
ii. SiG
iii. SiGG'
iv. 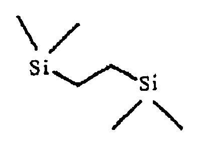
v. 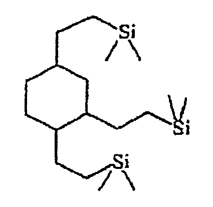
vi. 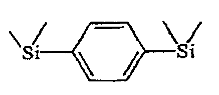
vii. 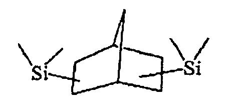
in which G and G' represent, independently of one another, an unbranched or branched aliphatic, cycloaliphatic or aromatic residue with 0 to 8 carbon atoms, in which one or more carbon atoms can be replaced by (C=O), O(C=O) or Si,
n and m represent, independently of one another, 0, 1, 2 or 3 and n + m amount to 2 to 6,
and in which the molar mass of the epoxide or the average molar mass of the mixture of epoxides is 250 to 1000 g/mol,
(b) 0 to 80 weight% of an epoxide or a mixture of epoxides which are different from (a),
(c) 30 to 85 weight% of fillers,
(d) 0.01 to 25 weight% of initiators, retarders and/or accelerators,
(e) 0 to 25 weight% of auxiliaries,
in which the percentages are in each case based on the total weight of the composition.

2. Polymerizable dental material according to Claim 1, comprising:
(a) 5 to 50 weight% of an epoxide or a mixture of epoxides of the general formula: 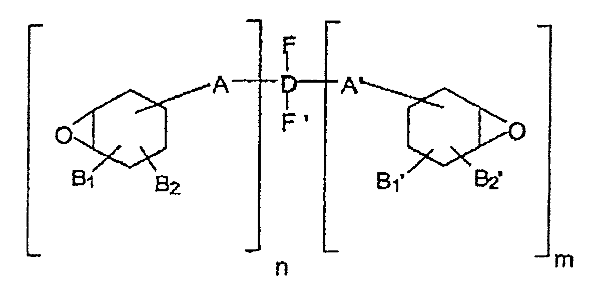 in which
A and A' represent, independently of one another, an unbranched or branched aliphatic, cycloaliphatic or aromatic residue with 0 to 10 carbon atoms, in which one or more carbon atoms can be replaced by C=O, O(C=O) or Si,
B1, B1', B2 and B2' represent, independently of one another, H or an unbranched or branched aliphatic residue with 0 to 4 carbon atoms, in which one or more carbon atoms can be replaced by O, (C=O), O(C=O) or Si,
F and F' represent, independently of one another, an unbranched or branched aliphatic, cycloaliphatic or aromatic residue with 0 to 10 carbon atoms, in which one or more carbon atoms can be replaced by (C=O), O(C=O) or Si,
D represents one of the following constituents, which is bonded to the constituents A and/or A' via the Si atom:
i. Si
ii. SiG
iii. SiGG'
iv. 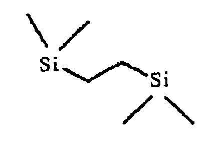
v. 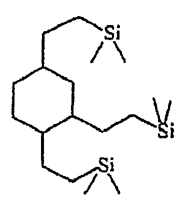
vi. 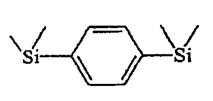
vii. 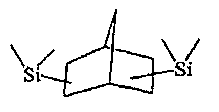
in which G and G' represent, independently of one another, an unbranched or branched aliphatic, cycloaliphatic or aromatic residue with 0 to 8 carbon atoms, in which one or more carbon atoms can be replaced by (C=O), O(C=O) or Si,
n and m represent, independently of one another, 0, 1 , 2 or 3 and n + m amount to 2 to 6,
and in which the molar mass of the epoxide or the average molar mass of the mixture of epoxides is 250 to 1000 g/mol,
(b) 0 to 60 weight% of an epoxide or a mixture of epoxides which are different from (a),
(c) 30 to 85 weight% of fillers,
(d) 0.01 to 20 weight% of initiators, retarders and/or accelerators,
(e) 0 to 25 weight% of auxiliaries,
in which the percentages are in each case based on the total weight of the composition.

3. Polymerizable dental material according to one of Claims 1 to 2, comprising, as component (a), one or more of the following epoxides: 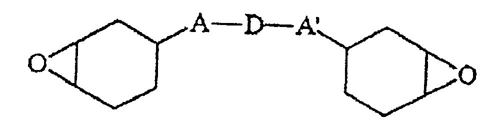 in which
A and A' represent, independently of one another, an aliphatic residue with 0 to 2 carbon atoms,
D represents SiGG',
G and G' represent, independently of one another, an unbranched or branched aliphatic or aromatic residue with 0 to 8 carbon atoms,
and in which the molar mass of the epoxide or the average molar mass of the mixture of epoxides is 250 to 500 g/mol.

4. Polymerizable dental material according to Claim 1 to 2, comprising, as component (a), one or more of the following epoxides:
i. Silane, methylbis[2-(7-oxabicyclo[4.1.0]hept-3-yl)ethyl]phenyl- 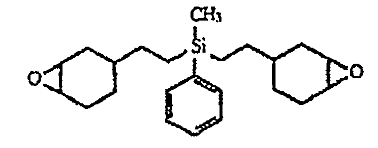
ii. Silane, dimethylbis[2-(7-oxabicyclo[4.1.0]hept-3-yl)methyl]- 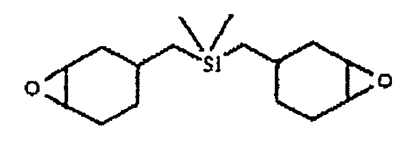
iii. Silane, dimethyl[2-(7-oxabicyclo[4.1.0]hept-3-yl)methyl][2-(7-oxabicyclo[4.1.0]hept-3-yl)ethyl]- 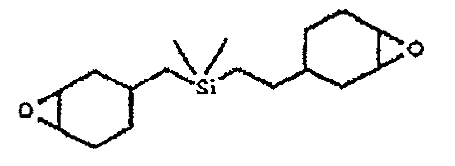
iv. Silane, 1,4-phenylenebis[dimethyl[2-(7-oxabicyclo[4.1.0]hept-3-yl)ethyl]]- 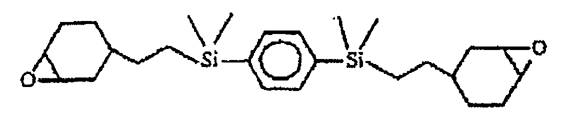
v. Silane, 1,2-ethylenebis[dimethyl[2-(7-oxabicyclo[4.1.0]hept-3-yl)ethyl]]- 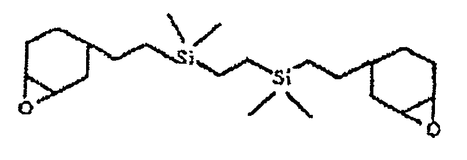
vi. Silane, dimethylbis[2-(7-oxabicyclo[4.1.0]hept-3-yl)ethyl]- 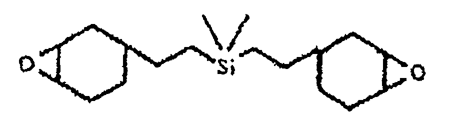
vii. Silane, 2,5-bicyclo[2.2.1]heptylenebis[dimethyl[2-(7-oxabicyclo[4.1.0]hept-3-yl)ethyl]]- 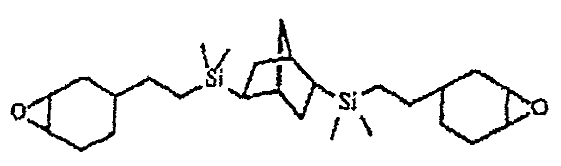
viii. Silane, 1,6-hexylenebis[dimethyf[2-(7-oxabicyclo[4.1.0]hept-3-yl)ethyl]]- 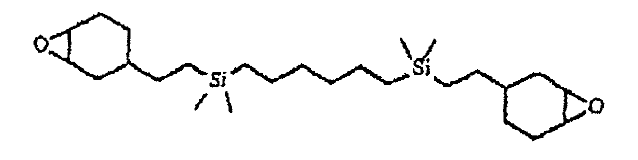
ix. Silane, 1,1',1"-(1,2,4-cyclohexanetriyltri-2,1-ethanediyl)tris[dimethyl[2-(7-oxabicyclo[4.1.0]hept-3-yl)ethyl]]-

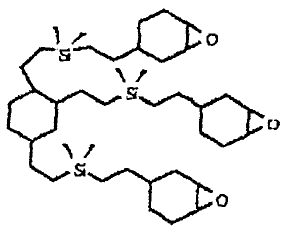

5. Polymerizable dental material according to one of Claims 1 to 4, comprising, as fillers according to component (c), quartz, ground glasses, silica gels and/or silicas, granulates thereof and/or ground plastics.

6. Polymerizable dental material according to one of Claims 1 to 5, comprising, as initiators, Lewis acids and/or Brönsted acids or compounds from which such acids arise by irradiation with UV light or visible light, pressure and/or heat or by chemical reaction.

7. Polymerizable dental material according to one of Claims 1 to 6, comprising, as auxiliaries, diluents, stabilizers, inhibitors and/or pigments.

8. Polymerizable dental material according to one of Claims 1 to 7, comprising
A at least one base paste, comprising epoxides or mixtures of epoxides of the components (a) and (b), a portion or the total amount of the fillers of the component (c), optionally retarders and/or accelerators according to component (d), and optionally auxiliaries of the component (e), and, spatially separated therefrom,
B at least one catalyst paste, comprising at least one initiator according to component (d), optionally a portion of the fillers of the component (c) and optionally auxiliaries according to component (e),
in which base and catalyst paste are reacted with one another to obtain the polymerizable dental material.

9. Container, in particular cartridge or mixing capsule, comprising a polymerizable dental material according to one of Claims 1 to 8.

10. Dispensing device, comprising a polymerizable dental material according to one of Claims 1 to 8.

## Revendications

1. Masse dentaire polymérisable contenant:
(a) 3 à 80% en poids d'un époxyde ou d'un mélange d'époxydes de formule générale: 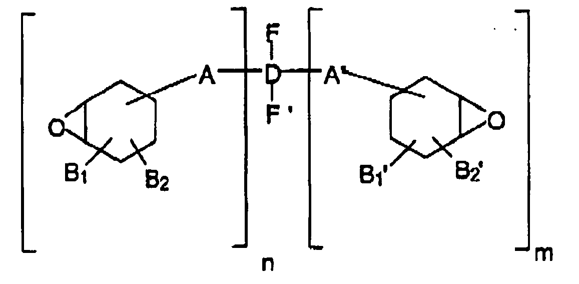 où
A, A' signifient, indépendamment l'un de l'autre, un radical non ramifié ou ramifié, aliphatique, cycloaliphatique ou aromatique comprenant 0 à 13 atomes de carbone, un ou plusieurs atomes de carbone pouvant être remplacés par C=O, O(C=O), Si, N, S,
B1, B1', B2, B2' signifient, indépendamment l'un de l'autre, H, un radical non ramifié ou ramifié, aliphatique, cycloaliphatique ou aromatique comprenant 0 à 6 atomes de carbone, un ou plusieurs atomes de carbone pouvant être remplacés par O, (C=O), O(C=O), Si, N, S,
F, F' signifient, indépendamment l'un de l'autre, un radical non ramifié ou ramifié, aliphatique, cycloaliphatique ou aromatique comprenant 0 à 10 atomes de carbone, un ou plusieurs atomes de carbone pouvant être remplacés par (C=O), O(C=O), Si, N, S,
D représente un des constituants suivants, qui est lié via l'atome de Si au constituant A et/ou A':
i. Si
ii. SiG
iii. SiGG'
iv. 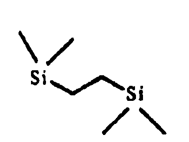
v. 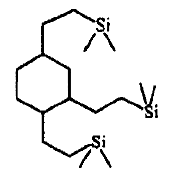
vi. 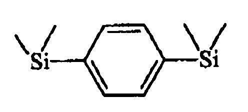
vii. 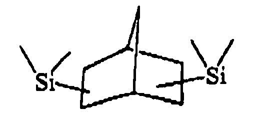
où
G, G' signifient, indépendamment l'un de l'autre, un radical non ramifié ou ramifié, aliphatique, cycloaliphatique ou aromatique comprenant 0 à 8 atomes de carbone, un ou plusieurs atomes de carbone pouvant être remplacés par (C=O), O(C=O), Si,
n et m signifient, indépendamment l'un de l'autre, 0, 1, 2 ou 3 et n+m vaut 2 à 6,
et où la masse molaire de l'époxyde ou la masse molaire moyenne du mélange des époxydes est de 250 à 1 000 g/mole,
(b) 0 à 80% en poids d'un époxyde ou d'un mélange d'époxydes, qui sont différents de (a),
(c) 30 à 85% en poids de charges,
(d) 0,01 à 25% en poids d'initiateurs, de retardateurs et/ou d'accélérateurs,
(e) 0 à 25% en poids d'adjuvants,
les indications en % se rapportant à chaque fois au poids total de la composition.

2. Masse dentaire polymérisable selon la revendication 1, contenant :
(a) 5 à 50% en poids d'un époxyde ou d'un mélange d'époxydes de formule générale: 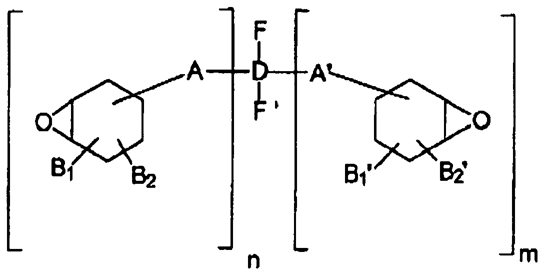 où
A, A' signifient, indépendamment l'un de l'autre, un radical non ramifié ou ramifié, aliphatique, cycloaliphatique ou aromatique comprenant 0 à 10 atomes de carbone, un ou plusieurs atomes de carbone pouvant être remplacés par C=O, O(C=O), Si,
B1, B1', B2, B2' signifient, indépendamment l'un de l'autre, H, un radical non ramifié ou ramifié, aliphatique, comprenant 0 à 4 atomes de carbone, un ou plusieurs atomes de carbone pouvant être remplacés par O, (C=O), O(C=O), Si,
F, F' signifient, indépendamment l'un de l'autre, un radical non ramifié ou ramifié, aliphatique, cycloaliphatique ou aromatique comprenant 0 à 10 atomes de carbone, un ou plusieurs atomes de carbone pouvant être remplacés par (C=O), O(C=O), Si,
D représente un des constituants suivants, qui est lié via l'atome de Si au constituant A et /ou A':
i. Si
ii. SiG
iii. SiGG'
iv. 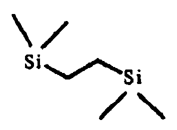
v. 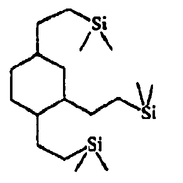
vi. 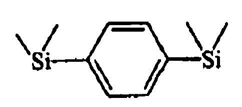
vii. 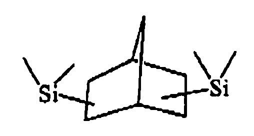
où
G, G' signifient, indépendamment l'un de l'autre, un radical non ramifié ou ramifié, aliphatique, cycloaliphatique ou aromatique comprenant 0 à 8 atomes de carbone, un ou plusieurs atomes de carbone pouvant être remplacés par (C=O), O(C=O), Si,
n et m signifient, indépendamment l'un de l'autre, 0, 1, 2 ou 3 et n+m vaut 2 à 6,
et où la masse molaire de l'époxyde ou la masse molaire moyenne du mélange d'époxydes est de 250 à 1 000 g/mole,
(b) 0 à 60% en poids d'un époxyde ou d'un mélange d'époxydes, qui sont différents de (a),
(c) 30 à 85% en poids de charges,
(d) 0,01 à 20% en poids d'initiateurs, de retardateurs et/ou d'accélérateurs,
(e) 0 à 25% en poids d'adjuvants,
les indications en % se rapportant à chaque fois au poids total de la composition.

3. Masse dentaire polymérisable selon la revendication 1 ou 2, contenant comme composant (a) un ou plusieurs des époxydes suivants: 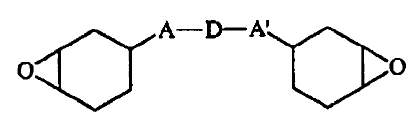 où
A, A' signifient, indépendamment l'un de l'autre, un radical aliphatique comprenant 0 à 2 atomes de carbone,
D signifie SiGG',
G, G' signifient, indépendamment l'un de l'autre, un radical non ramifié ou ramifié, aliphatique ou aromatique comprenant 0 à 8 atomes de carbone, et où la masse molaire de l'époxyde ou la masse molaire moyenne du mélange d'époxydes est de 250 à 500 g/mole.

4. Masse dentaire polymérisable selon les revendications 1 à 2, contenant comme composant (a) un ou plusieurs des époxydes suivants:
i. Silanes, méthylbis[2-(7-oxabicyclo[4.1.0]hept-3-yl)éthyl]phényl- 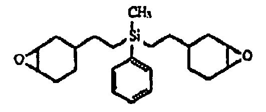
ii. Silanes, diméthylbis[2-(7-oxabicyclo[4.1.0]hept-3-yl)méthyl]- 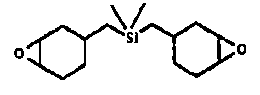
iii. Silanes, diméthyl[2-(7-oxabicyclo[4.1.0]hept-3-yl)méthyl][2-(7-oxabicyclo[4.1.0]hept-3-yl)éthyl]- 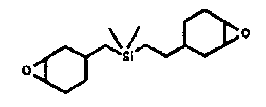
iv. Silanes, 1,4-phénylènebis[diméthyl[2-(7-oxabicyclo[4.1.0]hept-3-yl)éthyl]]- 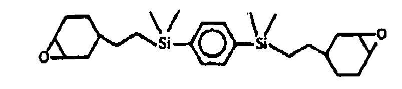
v. Silanes, 1,2-éthylènebis[diméthyl[2-(7- oxabicyclo[4.1.0]hept-3-yl)éthyl]]- 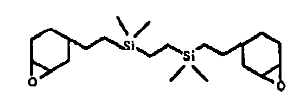
vi. Silanes, diméthylbis[2-(7-oxabicyclo[4.1.0]hept-3-yl)éthyl]- 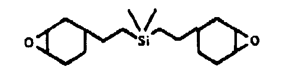
vii. Silanes, 2,5-bicyclo[2.2.1.]heptylènebis[diméthyl[2-(7-oxabicyclo[4.1.0]hept-3-yl)éthyl]]- 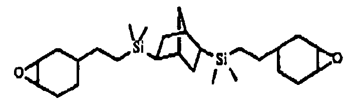
viii. Silanes, 1,6-hexylènebis[diméthyl[2-(7-oxabicyclo[4.1.0]hept-3-yl)éthyl]]- 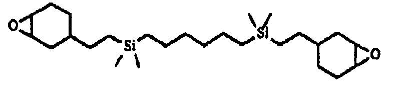
ix. Silane, 1,1',1"-(1,2,4-cyclohexanetriyltri-2,1-éthanediyl)tris(diméthyl[2-(7-oxabicyclo[4.1.0]hept-3-yl)éthyl]]-

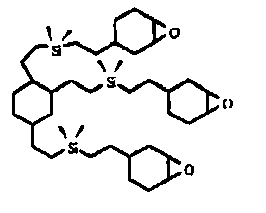

5. Masse dentaire polymérisable selon l'une quelconque des revendications 1 à 4, contenant comme charges selon le composant (c) du quartz, des verres broyés, des gels siliciques et/ou des silices, leurs granulats et/ou des matériaux synthétiques broyés.

6. Masse dentaire polymérisable selon l'une quelconque des revendications 1 à 5, contenant comme initiateurs des acides de Lewis et/ou de Brönstedt ou des composés à partir desquels ces acides sont formés par irradiation avec de la lumière UV, de la lumière visible, sous l'effet de la pression et/ou de chaleur ou par une réaction chimique.

7. Masse dentaire polymérisable selon l'une quelconque des revendications 1 à 6, contenant comme adjuvants des diluants, des stabilisateurs, des inhibiteurs et/ou des pigments.

8. Masse dentaire polymérisable selon l'une quelconque des revendications 1 à 7, contenant
A. au moins une pâte de base, contenant des époxydes ou des mélanges d'époxydes des composants (a) et (b), une partie ou la proportion totale de charges du composant (c), le cas échéant des retardateurs et/ou des accélérateurs selon le composant (d) et le cas échéant des adjuvants des composants (e), ainsi que, de manière séparée dans l'espace de celle-ci,
B. au moins une pâte de catalyseur, contenant au moins un initiateur selon le composant (d), le cas échéant une partie des charges du composant (c) et le cas échéant des adjuvants selon le composant (e), obtenue en faisant réagir la pâte de base et la pâte de catalyseur pour obtenir la masse dentaire polymérisable.

9. Récipient, en particulier cartouche ou capsule de mélange contenant une masse dentaire polymérisable selon l'une quelconque des revendications 1 à 8.

10. Dispositif d'expulsion, contenant une masse dentaire polymérisable selon l'une quelconque des revendications 1 à 8.
